Europäisches Patentamt

European Patent Office

Office européen des brevets

(11) Publication number: **0 226 278**
**A1**

## (12) EUROPEAN PATENT APPLICATION

(21) Application number: 86307451.4

(22) Date of filing: 29.09.86

(51) Int. Cl.⁴: **A 61 F 2/64**

(30) Priority: 30.09.85 GB 8524028

(43) Date of publication of application:
24.06.87 Bulletin 87/26

(84) Designated Contracting States:
DE FR

(71) Applicant: J.E. HANGER & COMPANY LIMITED
Roehampton Lane
Roehampton London SW15 5PL(GB)

(72) Inventor: May, Denis Ronald William
Barons Mead Wayneflete Tower Avenue
Esher Surrey KT10 8QG(GB)

(74) Representative: Cole, Paul Gilbert et al,
HUGHES CLARK & CO. 63 Lincoln's Inn Fields
London WC2A 3JU(GB)

(54) Leg prosthesis and releasable locking mechanism therefor.

(57) A rotator for connecting a stump socket to lower parts of an artificial leg comprises upper and lower plates (12, 26) journalled together to permit rotation of lower parts (24) from a walking attitude about the longitudinal direction of the leg so that the user can sit cross-legged on the ground. A buckle member (41) fits between the plates (12, 26) so as to be slideable to and fro along a direction substantially at right angles to the longitudinal direction of the leg to engage or disengage an abbreviated offset tongue (42) carried by the buckle member (41) with a recess (21) in the upper plate (12). It is an advantage of the rotator that it occupies only a small distance longitudinally of the leg.

FIG.3.

## LEG PROSTHESIS AND RELEASABLE LOCKING MECHANISM THEREFOR

This invention relates to a leg prosthesis and to a releasably locking swivel mechanism therefor.

Broadly stated, the invention provides a leg prosthesis wherein a stump socket is connected to lower parts of the leg through a releasable swivel permitting rotation of the lower parts from a walking attitude about the longitudinal direction of the leg so that the user can sit cross-legged on the ground.

The invention further provides a releasable swivel mechanism for a leg prosthesis for interposition between first and second parts of a prosthetic leg to permit relative rotation about a longitudinal axis from a working position, said mechanism comprising first and second plate members for attachment to the first and second parts of the leg secured together in face to face contact with interfitting means defining a journal bearing for said members, lateral portions of the first member being removed to locate a buckle member slideably between the first and second plates, the buckle member having an abbreviated offset tongue that travels over the periphery of the second member until it encounters a recess therein into which it is snap engaged by resilient means to define the working position.

The swivel mechanism can be fitted between a socket alignment device and lower portions such as a knee member of an artificial leg and has the advantages that it is simple to fit but robust and occupies only a minimal axial length.

An embodiment of the invention will now be described, by way of example only, with reference to the accompanying drawings, in which:-

Figure 1 is a plan view of a lower section of a swivel mechanism according to the invention in position on the upper part of an artificial knee with a socket alignment device removed to expose an upper section of the mechanism;

Figure 2 is an exploded view of parts of the lower section of the swivel mechanism;

Figure 3 is a diagrammatic, partly-sectioned front view of the swivel mechanism; and

Figures 4 and 5 are diagrammatic sections of the swivel mechanism on the line Y-Y of Figure 3 in normal and rotated positions, respectively.

Figures 6 and 7 are respectively a diagrammatic perspective view and an exploded view of a second embodiment of a rotator according to the invention.

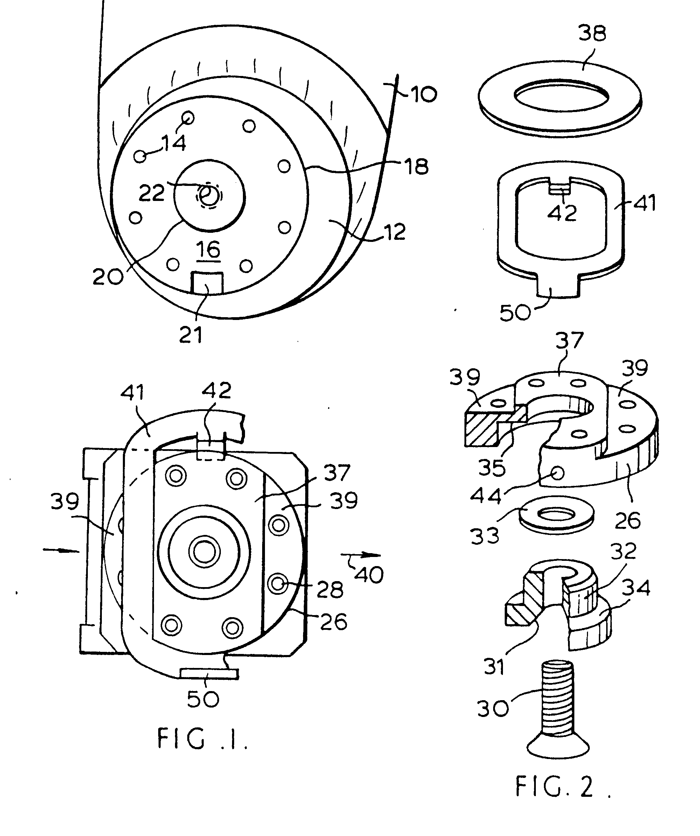

In Figure 1, a connector cup 10 providing an attachment and alignment function for a socket of a leg prosthesis has a round mounting plate 12 defining a first section of a releasable swivel and attached thereto by means of rivets 14 in a generally octagonal disposition. The lower face of the plate 12 has a downwardly projecting region defining one swivel plate 16 and bounded by an inscribed circle 18 on the plate 12 so that the centre of the plate 16 is offset from that of the plate 12. A stub shaft 20 is defined by an annulus on the lower face of the plate 16 and concentric therewith, and a threaded bore 22 in plate 12 is formed at the centre of the plate 16. The plate 16 has a smooth rim except for a single cut-out defining a socket 21.

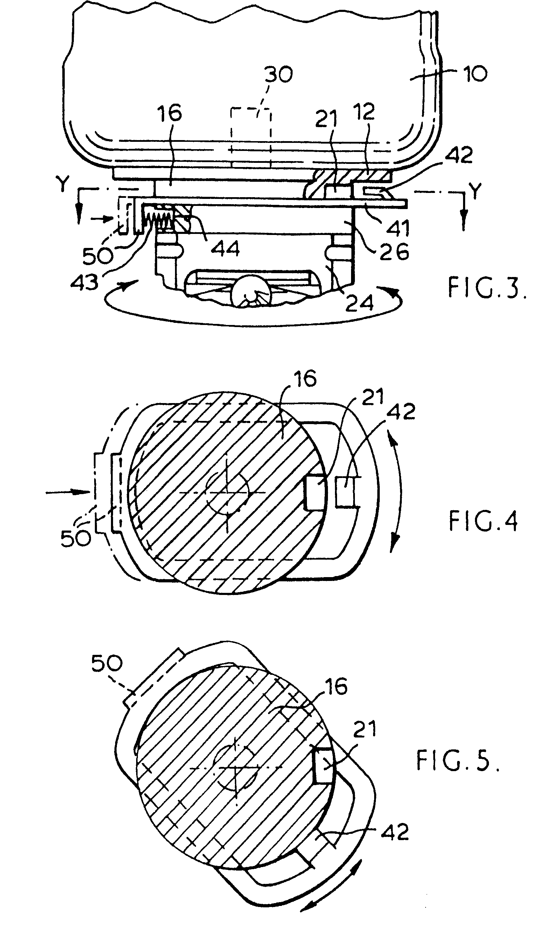

An annular plate 26 is attached to a top plate 24 of a prosthetic knee by means of mounting bolts 28 disposed in an octagonal pattern and defines a second section of the swivel. Before that attachment is made, an upstanding clamping bolt 30 (Figure 2) is fitted to a countersunk bore 31 of a flanged collar 32 with a washer 33 of PTFE or other low-friction material between the collar flange 34 and a rebate 35 in the lower face of the plate 26. Thereby, the collar 32 is supported for rotation with the stud 30 relative to the member 26 so that the bolt 30 does not work loose as the swivel is used. The top face 37 of the plate 26 abuts against the plate 16 with an interposed washer 38, again of PTFE or other low-friction material. The face 37 is bounded by a pair of opposed rebated segments 39 which are directed transversely of the anterior-posterior bore 40 and define a slideway for a slideable buckle member 41 that fits flush with the top face 37 between the plates 16, 26. An abbreviated upwardly-offset tongue 42 at one end of the member 41 rides around the rim of the plate 16 (Figures 4 and 5). The other end of the member 41 is formed with a depending handle 50, and a coil spring 43 located in a bore 44 in the rim of plate 26 is in compression against the handle 50 to urge the member 41 towards its locking position (chain-dotted lines). Thus when the tongue 42 registers with the socket 21 it snap-engages therewith to hold lower parts of the leg in the correct position for walking.

For assembly of the locking mechanism, the sleeve 32 and washer 33 are placed onto the bolt 30 and together positioned under the plate 26 which is then bolted in position on the knee top plate 24. Spring 43 is placed in the recess 44, after which buckle member 41 is positioned on plate 26 and the washer 38 is placed on top. The cup 10 with the pre-attached plate 12 is then lowered into place with the stub shaft 20 passing through washer 38 to between plate 39 and the cylindrical sidewall of collar 32. The bolt 30 is then located in the bore 22 and tightened by an Alum key or other suitable means through an access hole in the plate 24. The resulting assembly locks the lower parts of the knee in a walking or normal sitting position until the transversely sliding buckle member 41 is actuated by finger pressure on handle 50, after which the lower parts of the leg can rotate to allow the wearer to take up a cross-legged sitting position.

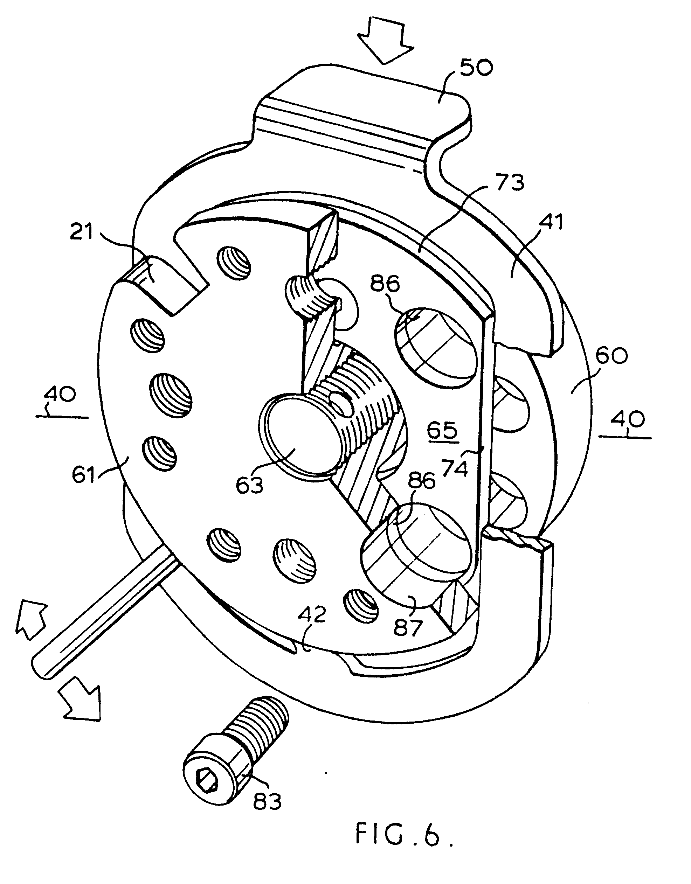

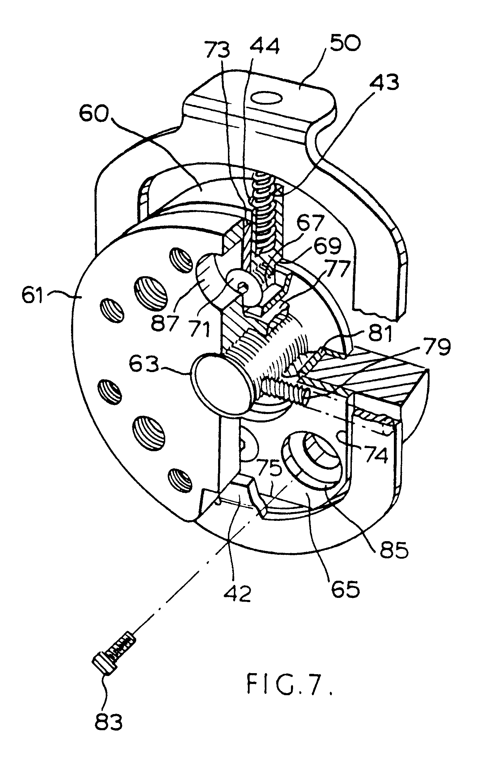

In Figures 6 and 7 which relate to a second embodiment of the invention first and second swivel plates 60, 61 are fastened together by clamping bolt 63 with locking plate or buckle member 41 intervening and with guide plate 65 of low friction material (e.g. Tuf-clad moly grade TCM 98) also intervening.

The first plate 60 is generally circular in plan with a central bore 67 from its inner face and a counterbore 69. It is provided with a pair of threaded holes for reception of countersunk fixing screws 71 of the guide plate 65. When viewed in plan the plate 65 has a curved end 73 conforming to the rim of the plate 60, straight sides 74 directed transversely of anterior-posterior line 40 to define a slideway for buckle member 41 and an inwardly positioned straight end 75. The assembly of plate 60 and guide plate 65 corresponds to the plate 26 of Figure 1. The second plate 61 is formed on its concealed face with an internally threaded bush 77 whose outer face acts as a stub axle and whose inner face is engaged by bolt 63 which is held in place by locking screw 79.

For assembly of the swivel mechanism, a flanged bush 81 of PTFE or other low-friction material is fitted into the counterbore or rebate 69 in the first swivel plate 60 and the clamping bolt 63 is inserted through the assembly so that the underside of its head contacts the flange of the bush 81 to provide a smooth swivel action. The buckle guide plate 65 is then fitted into place followed by buckle 41 with the spring 43 in bore 44 of plate 60 acting against the handle 50 of buckle 41. Plate 61 is then attached by engagement of clamping bolt 63, which protrudes through previously assembled bush 81 and plate 60, into the threaded bore in bush 77 by means of an allen key which fits into a socket in the head of bolt 63 and enables the assembly to be tightened to a required level of torque, after which locking screw 79 is tightened to prevent bolt 63 from working loose. The outer face of spigot 77 fits into the bush 81 which provides a low friction bearing. As in the previous embodiment, abbreviated offset tongue 42 at one end of buckle member 41 rides around the rim of plate 61 until its registers with socket 21 into which it is then snap-engaged by spring 43 to hold lower parts of the leg in the correct position for walking.

As will be seen from Figures 6 and 7, plates 60, 61 are provided with threaded through holes disposed in patterns so that upper and lower parts of the leg may be attached by means of cap head screws 83. The screws 83 are used either in a 4-screw or 8-screw configuration, and when the 4-screw configuration is used, the screws 83 are inserted into countersunk region 85 in the blind face of plate 60, the guide plate 65 being apertured at 86 correspondingly. The plate 61 is formed with a through-hole 87 that may be rotated into register with each region 85 of a set of holes in turn as the plate 61 rotates to give screw access thereto.

CLAIMS:

1. A leg prosthesis wherein a stump socket (10) is connected to lower parts (24) of the leg through a swivel permitting rotation of the lower parts (20) from a walking attitude about the longitudinal direction of the leg so that the user can sit cross-legged on the ground, characterised in that the swivel is releaseably lockable by catch means (21, 42) having a line of action substantially at right angles to the longitudinal direction of the leg.

2. A prosthesis according to Claim 1, wherein the swivel comprises first and second members (16, 26) respectively secured between a stump socket (10) and lower parts (24) of the limb and secured together in face to face contact with interfitting means (20, 32, 39) defining a journal bearing for said members. (16, 26).

3. A prosthesis according to Claim 2, wherein lateral portions (39) of the first member (26) are recessed to locate a buckle member (41) slideably between the first and second members (16, 26) the buckle member (41) having an abbreviated offset tongue (42) that travels over the periphery (18) of the second member (16) until it encounters a recess (21) therein into which it is snap-engaged by resilient means (43) to define the walking attitude.

4. A prosthesis according to Claim 3, wherein upstanding portions of said buckle member (41) opposite to said tongue (42) define a handle (50) by which said buckle (41) may be slid against the resistance of a coil spring (43) in compression between said handle (50) and a recess in said first member (26).

5. A prosthesis according to Claim 3 or 4, wherein the second member (11) has a projecting stub shaft (20) that fits into a hole in the first member (26), engaging a flanged collar (32) that fits into the hole from a side of the first member (26) remote from the second member (16), a connecting bolt (30) passing through the collar (32) and first member (26) and fastening into a threaded bore (22) in the second member (16).

6.    A prosthesis according to Claim 5, wherein a first washer (33) of low-friction material is located between the collar flange (34) and the first member (26) and a second washer (38) of low-friction material is located between the first and second members (16, 26).

7.    A prosthesis according to Claim 6, for an above-knee amputee wherein the mounting is fitted between socket connecting means and a knee joint.

8.    A releasable swivel mechanism for a leg prosthesis for interposition between first and second parts (10, 24) of a prosthetic leg to permit relative rotation about a longitudinal axis from a working position, said mechanism comprising first and second plate members (16, 26) for attachment to the first and second parts (10, 24) of the leg secured together in face to face contact with interfitting means (20, 32, 39) defining a journal bearing for said members (16, 26), characterised in that lateral portions (39) of the first member (26) are removed to locate a buckle member (41) slideably between the first and second plates (16, 26), the buckle member (41) having an abbreviated offset tongue (42) that travels over the periphery (18) of the second member (16) until it encounters a recess (21) therein into which it is snap-engaged by resilient means (43) to define the working position.

9.    A mechanism according to Claim 8, wherein the second member (16) has a projecting annulus (20) defining a stub shaft that fits into a hole in the first member (20), engaging a flanged collar (32) that fits into the hole from a side of the first member (26) remote from the second member (16), a connecting bolt (30) passing through the collar (32) and first member (26) and fastening into a threaded bore (22) in the second member (16).

10.    A mechanism according to Claim 9, wherein a first washer (33) of low-friction material is located between the collar flange (34) and the first member (26) and a second washer (38) of low-friction material is located between the first and second members (16, 26).

1—4

0226278

FIG .I.

FIG. 2 .

FIG.3.

FIG.4

FIG.5.

FIG.6.

FIG.7.

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| Y | DE-U-8 122 676 (SHEN-JONG) * figures 1, 3, 4; page 10, lines 13-26 * | 1,8 | A 61 F 2/64 |
| Y | US-A-3 351 955 (MIDDLETON) * claim 1; figure 1 * | 1,8 | |
| Y | ENGINEERING IN MEDICINE, vol. 10, no. 3, July 1981, pages 155-158, Whitstable, Kent, GB; K.K. CHAUDHRY et al.: "Modular concept in a multi-functional above knee prosthesis" * figures 1, 2 * | 1,8 | |
| A | US-A-4 520 512 (LEHNEIS) * claim 1; figures 4, 5 * | 1,2,8 | TECHNICAL FIELDS SEARCHED (Int Cl 4) |
| A | US-A-3 798 680 (PROUT) * figures 3-6 * | 1 | A 61 F 2/00 |

The present search report has been drawn up for all claims

| Place of search | Date of completion of the search | Examiner |
|---|---|---|
| BERLIN | 17-12-1986 | KANAL P K |